# EUROPEAN PATENT SPECIFICATION

(11) **EP 4 667 034 B1**
(45) Date of publication and mention of the grant of the patent: **02.12.2026**
(21) Application number: 25181949.6
(22) Date of filing: 11.06.2025
(51) Int. Cl.: A61M 15/00, A61M 11/00, B05B 17/00

(54) **ATOMIZER**
ZERSTÄUBER
ATOMISEUR

(30) Priority: 20.06.2024 CN 202421409839 U
(43) Date of publication of application: 24.12.2025
(73) Proprietor: Innovate (Wuxi) Medical Technology Co., Ltd, Wuxi City, Jiangsu Province (CN)
(72) Inventor: DING, Qi, Wuxi City, Jiangsu Province (CN); ZU, Xueqing, Wuxi City, Jiangsu Province (CN); HU, Chunxiao, Wuxi City, Jiangsu Province (CN); WU, Bo, Wuxi City, Jiangsu Province (CN); LI, Xiaoshan, Wuxi City, Jiangsu Province (CN); GAO, Hong, Wuxi City, Jiangsu Province (CN)
(74) Representative: Tahtadjiev, Konstantin

(56) References cited:
- WO-A1-2017/076590
- WO-A1-2022/019576
- WO-A1-2023/202334
- CN-A- 117 942 458
- CN-U- 216 798 421
- DE-A1- 102024 124 474
- JP-A- 2015 062 892
- US-A1- 2017 072 085
- US-B1- 9 211 980

## Description

### FIELD OF INVENTION

The present disclosure relates to atomizer, and more particularly to a core-replaceable atomizer.

### DESCRIPTION OF RELATED ARTS

A medical nebulizer atomizes the liquid medicine and then enters the patient's body through the respiratory tract, thus achieving the purpose of atomization treatment.

The liquid medicine in the current nebulizer relies on gravity to contact the atomizer sheet in the nebulizer, which means that there are requirements for the placement of the nebulizer, such as position and angle. That is, the nebulizer needs to be placed in a standing position. Once it is inverted and deviated, the nebulizer will not work properly.

At the same time, in actual use, some patients need more than one medicine for nebulization treatment. For example, upper respiratory tract infection requires medicines such as budesonide or ambroxol. If there is subsequent inflammation, additional dextromethorphan or pentoxyverine cough suppressant may be needed for treatment. If there is sputum, cholinergics may also be needed.

Different medicines need to be used separately to achieve different treatment purposes (some hospitals will require that the nebulizer be replaced entirely). In this process, apart from the cost and cumbersome operation, the removal of the nebulizer itself will cause the breathing circuit to be interrupted for a long time, which may even affect the patient's life safety, especially in the ICU or hyperbaric oxygen chamber. There is a potential risk of cross-infection of bacteria (especially super bacteria). Once infected, the consequences are extremely serious.

In addition, the conventional nebulizers need to be reused many times due to cost and other reasons, but cleaning and disinfection are cumbersome. If the cleaning is not thorough, the mixing of different medicines will also affect the safe use of the medicines themselves.

If there is no filter element in the medicine storage device, impurities generated during the medicine preparation process, such as glass ampoules or vial plugs, can easily cause the mesh pores of the nebulizer to become clogged, affecting the normal atomization of the nebulizer and even shortening its service life.

DE102024124474A1 has disclosed an aerosol generation device comprising a container with a tank formed therein for storing a liquid to be sprayed. The container has an outlet opening, an ultrasonic generator by means of which the aerosol can be generated from the liquid, a mouthpiece attached to the container adjacent to its outlet opening, which has a discharge opening for releasing the aerosol generated by the ultrasonic generator, and a wick arranged in the outlet opening of the container for transporting the liquid from the tank to the ultrasonic generator. The ultrasonic generator comprises a first part with a piezoelectric element and a second part with a plurality of passages through which the liquid can pass. Here, the outlet end of the wick is spaced so far away from the second part of the ultrasonic generator that the vibrating second part with the multitude of passages only touches or immerses in the liquid which has accumulated at the outlet end of the wick due to the surface tension of the same liquid, and thereby the liquid is absorbed in the passages of the second part of the ultrasonic generator.

US9211980B1 has disclosed a microfluidic refill including a reservoir having a hollow body and an opening; a transport member in fluid communication with the reservoir; and a lid enclosing the opening of the reservoir. The lid is in fluid communication with the transport member. The lid comprises a rigid microfluidic delivery member. The rigid microfluidic delivery member includes a die and electrical traces that are in electrical communication with the die, wherein the electrical traces terminate at electrical contacts, wherein the electrical traces are disposed on only one plane. The die has a fluid chamber in fluid communication with the transport member at an inlet of the fluid chamber and with an orifice at an outlet of the fluid chamber.

CN117942458A1 has disclosed a portable atomizer comprising a main body assembly, a liquid suction part, an atomizing part and a plugging part, the main body assembly is provided with a liquid storage cavity and an atomizing cavity, the liquid storage cavity is provided with an air inlet hole, the atomizing cavity is provided with a mist outlet, one end of the liquid suction part is contained in the liquid storage cavity, and the other end of the liquid suction part is contained in the atomizing cavity; the liquid suction part is used for sucking liquid medicine in the liquid storage cavity into the main body assembly, the atomization part is contained in the atomization cavity and electrically connected with the main body assembly, the atomization part is used for sucking the liquid medicine from the liquid storage cavity through the liquid suction part and atomizing and releasing the liquid medicine, and the plugging part is arranged in the liquid storage cavity and covers the air inlet hole. The plugging piece can prevent liquid medicine in the liquid storage cavity from flowing out of the air inlet hole, and air can enter the liquid storage cavity through the air inlet hole, so that by arranging the air inlet hole and the plugging piece, the air pressure in the liquid storage cavity is balanced with the external atmospheric pressure, the liquid medicine can be conveniently sucked by the liquid suction piece and conveyed to the atomization cavity, and the mist outlet amount of the portable atomization cavity is guaranteed; therefore, symptoms can be effectively relieved and illness can be effectively treated.

US2017/072085A1 has disclosed a cartridge for a microfluidic delivery system. The cartridge includes a reservoir for containing a fluid composition. The cartridge includes a capillary tube having a first end portion and a second end portion opposing the first end portion. The first end portion of the capillary tube is in fluid communication with the reservoir. The second end portion of the capillary tube is operatively connected with the reservoir. The cartridge includes a restriction member that is in fluid communication with the capillary tube and that is configured to restrict fluid flow. The cartridge comprises a microfluidic delivery member connected with and in fluid communication with the reservoir, wherein the microfluidic delivery member comprises a die having at least one nozzle.

JP2015062892A has disclosed that to provide an ultrasonic atomizer having excellent spraying stability.A solution is that when a liquid container is attached/detached to/from an ultrasonic atomizer, an absorber is also attached/detached to/from the ultrasonic atomizer together with the liquid container. A diaphragm vibrates so that the liquid supplied by the absorber is atomized and sprayed. The absorber has a liquid-holding weight of 54 mg or more and 86 mg or less.

CN216798421U has disclosed a medicament atomizer comprising a liquid medicine storage cabin and an atomizing rod, the atomizing rod is filled with the liquid medicine storage cabin, one end of the liquid medicine storage cabin is sealed, the other end of the liquid medicine storage cabin is sealed by a piezoelectric ceramic atomizing sheet, the other side of the piezoelectric ceramic atomizing sheet is connected with a mist guide nozzle, an electrode is arranged outside a cabin body, a filler cabin is arranged at one end of the atomizing rod, and the filler cabin is connected with the liquid medicine storage cabin. One end, provided with an electrode, of the liquid medicine storage cabin is butted with the filler cabin, and the electrode of the liquid medicine storage cabin is butted with an electrode conduction battery of the atomizing rod to supply power; an air inlet is formed in the other end of the cylindrical shell, a circulating air path is formed between the air inlet and the filler bin, and an air flow sensor is arranged at the air inlet. According to the medicine atomizer, the medicine liquid storage cabin for independently packaging the medicine liquid is adopted, the independent medicine liquid storage cabin is in butt joint with the atomizer, atomization can be carried out, a user does not need to add the medicine liquid by himself, the whole medicine liquid storage cabin is replaced after a single medicine liquid is used, the problems of medicine pollution and inaccurate quantification are solved, and the medicine atomizer is convenient to use, safe, simple and convenient.

WO2017/076590A1 has disclosed an aerosol-generating system, a cartridge for an aerosol- generating system, an atomiser for an aerosol-generating system and a method of generating an aerosol using the aerosol- generating system, wherein the system comprises a liquid-storage portion comprising a housing for holding a liquid aerosol- forming substrate; heating means arranged to heat liquid aerosol- forming substrate; a vibratable element comprising a plurality of passages through which heated liquid aerosol-forming substrate passes to form an aerosol; and an actuator arranged to vibrate the vibratable element to generate the aerosol.

WO2022/019576A1 has disclosed an ultrasound-based aerosol generating device having a novel structure capable of reducing cartridge replacement costs and a control method therefor are provided. An ultrasound-based aerosol generating device according to several embodiments of the present disclosure may comprise a replaceable cartridge for storing a liquid aerosol forming material and a control body coupled to the cartridge. In addition, the control body may comprise a control part and a vibration member for generating ultrasonic vibration so as to vaporize the aerosol forming material stored in the cartridge. At this time, the control part may monitor the temperature of the vibration member and control an operation of the vibration member on the basis of a monitoring result.

WO2023/202334A1 has disclsoed an atomizer and an aerosol generating device. The atomizer comprises a liquid storage member, an ultrasonic atomization sheet, a liquid guide member, and an elastic member. In the atomizer structure, the elastic member is provided in the liquid storage member, a second end of the liquid guide member is supported on the elastic member, and the elastic member is used to abut against the liquid guide member towards the ultrasonic atomization sheet, that is, when the ultrasonic atomization sheet is in a vibration operation mode, a first end of the liquid guide member is always in contact with the ultrasonic atomization sheet, so as to effectively prevent poor contact or an excessive assembly gap between the liquid guide member and the ultrasonic atomization sheet, thereby ensuring that the liquid guide member can continuously and stably transmit an aerosol-forming matrix to the ultrasonic atomization sheet, thus facilitating improving the atomization stability and atomization effect of the ultrasonic atomization sheet. In addition, because the elastic member can abut against the liquid guide member towards the ultrasonic atomization sheet, the situation in which the liquid guide member and the ultrasonic atomization sheet are assembled too tightly can also be prevented from affecting the normal operation of the ultrasonic atomization sheet.

### SUMMARY OF THE PRESENT INVENTION

The invention is set out in the appended set of claims 1-2.

### BRIEF DESCRIPTION OF THE DRAWINGS

Fig. 1 is a perspective view of a core-replaceable atomizer according to a first preferred embodiment of the present disclosure, which is considered as useful for understanding the invention.
Fig. 2 is a sectional view of the core-replaceable atomizer according to the first preferred embodiment of the present disclosure, which is considered as useful for understanding the invention.
Fig. 3 is a schematic view illustrating an atomization generating assembly of the core-replaceable atomizer according to the first preferred embodiment of the present disclosure, which is considered as useful for understanding the invention.
Fig. 4 is an exploded view of illustrating the atomization generating assembly of the core-replaceable atomizer according to the first preferred embodiment of the present disclosure, which is considered as useful for understanding the invention.
Fig. 5 is a perspective view of an atomizer according to a second preferred embodiment of the present disclosure, which is considered as useful for understanding the invention.
Fig. 6 is a sectional view of the atomizer according to the second preferred embodiment of the present disclosure, which is considered as useful for understanding the invention.
Fig. 7 is an exploded view of the atomizer according to the second preferred embodiment of the present disclosure, which is considered as useful for understanding the invention.
Fig. 8 is a perspective view of an atomizer according to a third preferred embodiment of the present invention.
Fig. 9 is an exploded view of the atomizer according to the third preferred embodiment of the present invention
Fig. 10 is a sectional view of the atomizer according to the third preferred embodiment of the present invention.
Fig. 11 is another sectional view of the atomizer according to the third preferred embodiment of the present invention.
Fig. 12 is an enlarged perspective view of a medicine storage assembly of the atomizer according to the third preferred embodiment of the present invention.
Fig. 13 is an enlarged sectional view of the medicine storage assembly of the atomizer according to the third preferred embodiment of the present invention.
Fig. 14 is an enlarged sectional view of an air exhaust structure of the medicine storage assembly of the atomizer according to the third preferred embodiment of the present invention.
Fig. 15 is a sectional view of the atomizer according to an alternative mode of the third preferred embodiment of the present invention.

### DETAILED DESCRIPTION OF THE PREFERRED EMBODIMENT

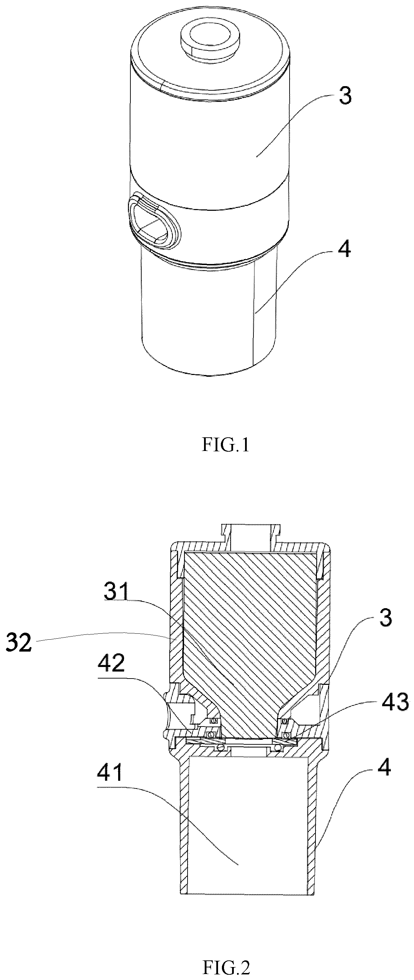

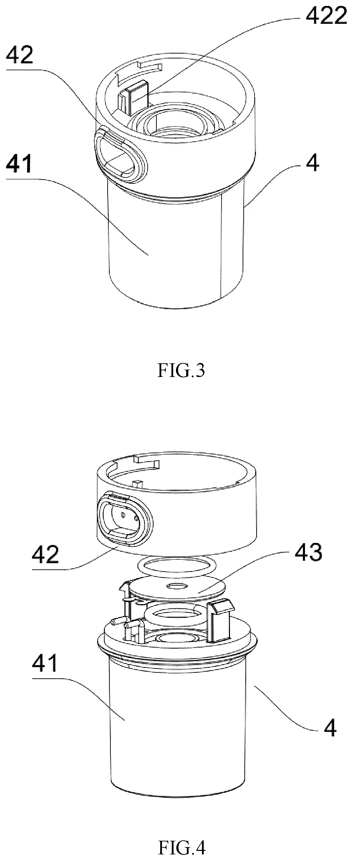

Referring to Figs. 1 to 4, a core-replaceable atomizer according to a first preferred embodiment of the present disclosure comprises a liquid medicine storage assembly 3 and an atomization generating assembly 4.

The liquid medicine storage assembly 3 has a built-in filter element 31 for guiding and filtering the liquid medicine, and one end of the atomization generating assembly 4 is connected to the liquid medicine storage assembly 3, and the other end is connected to the patient's inhalation end.

The atomization generating assembly 4 comprises a base 41 for supporting the atomizing element 43 and a connecting tube 42 connected to the patient's inhalation end. One end of the base 41 is connected to the liquid medicine storage assembly 3, and the other end is provided with an atomization area for placing the atomizing element 43. One end of the connecting tube 42 is fixed by connecting to the base 41 to limit the atomizing element 43, and the other end is an interface connected to the patient's inhalation end.

After the device is connected to the breathing circuit, the atomizing element 43 in the atomization generating assembly 4 atomizes the liquid medicine in the liquid medicine storage assembly 3 and then inputs it into the breathing circuit.

Before use, it is necessary to add liquid medicine into the liquid medicine storage assembly 3, then connect it to the breathing circuit, and finally connect the atomizing element 43 to the power supply before it can be turned on for use.

When in use, one end of the filter element 31 for adsorbing the medicine liquid will be in contact with the atomization generating assembly 4, and the medicine liquid will be continuously atomized by the atomization generating assembly 4.

In order to ensure the fit between the filter element 31 and the atomization generating assembly 4 and prevent the filter element 31 from shaking in the liquid medicine storage assembly 3, the size of the filter element 31 is normally larger than the size of the inner cavity of the liquid medicine storage assembly 3, that is, the filter element 31 and the inner cavity fixing structure of the liquid medicine storage assembly 3 are interference fit.

When the medicine needs to be replaced, the liquid medicine storage assembly 3 can be directly replaced, and the atomization generating assembly 4 can be left in the connection of the breathing circuit.

In order to facilitate the filling of medicine, the liquid medicine storage assembly 3 comprises a shell 32 connected and fixed to the base 41. The rear side of the shell 32 is provided with a medicine injection hole, and a sealing cover is provided at the medicine injection hole.

Similarly, as shown in Figs. 1 to 4, the core-replaceable atomizer of the present solution can also be designed to comprise the detachably connected liquid medicine storage assembly 3 and the atomization generating assembly 4, and the atomized medicine can be replaced by simply replacing the liquid medicine storage assembly 3, eliminating the cleaning and disinfection process of the iquid medicine storage assembly 3, and is easy to operate. The medicine can also be prepared in advance, thereby improving the work efficiency of medical staff and reducing cross infection in the hospital. The relevant structure is as follows.

The liquid medicine storage assembly 3 and the atomization generating assembly 4 are detachably connected, and a plurality of groups of detachable fixing structures are arranged at the connection between the shell 32 of the liquid medicine storage assembly 3 and the base 41 of the atomization generating assembly 4. Each group of the fixing structures includes an "L"-shaped adapter block located on the shell 32 and an embedding area located on the base 41. The positions of the adapter block and the embedding area in each group of fixing structures correspond to each other. When installing or disassembling and replacing the liquid medicine storage assembly 3, the adapter block is attached to the top of the base 41, and is turned into or out of the embedding area by corresponding vertical pressure rotation to complete the installation or disassembly of the liquid medicine storage assembly 3.

In order to better fix the atomizing element 43 in the atomization generating assembly, a plurality of embedding legs 422 are provided at equal angles on one side of the connecting tube 42 close to the base 41, and are fixed after passing through the base 41. The atomizing element 43 is connected to an external power source through a connection interface on the side wall of the base 41.

In the compensation of the above two solutions, the atomizing element 43 adopts an atomizing sheet with pores, and sealing rings are arranged on the upper and lower sides of the atomizing element 43.

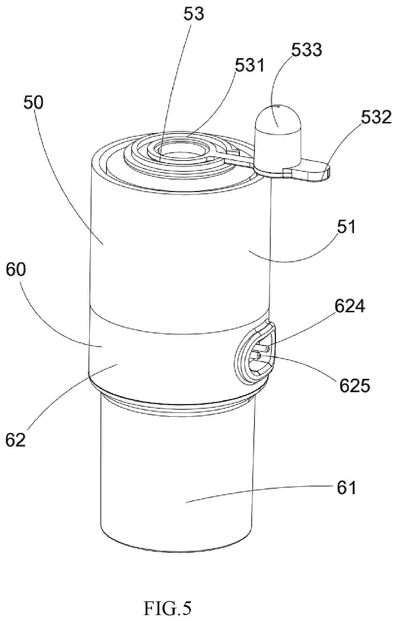

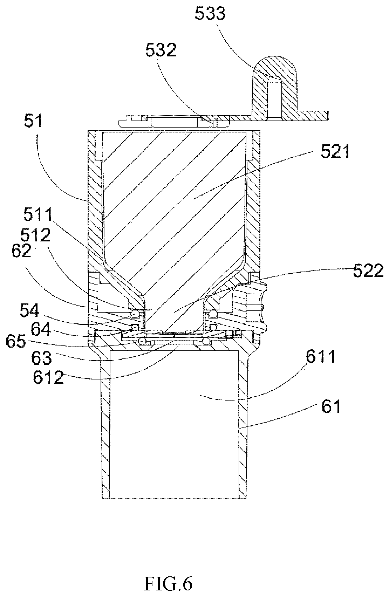

Referring to Fig. 5 to Fig. 7 of the drawings, an atomizer according to a second preferred embodiment of the present disclosure is illustrated, the atomizer comprises a medicine storage assembly 50 and an atomization assembly 60 for atomizing the medicine liquid delivered from the medicine storage assembly 50.

The medicine storage assembly 50 comprises an outer housing 51 having a storage cavity 511, a medicine liquid transporting element 52 disposed in the outer housing 51, and a medicine injection structure 53 mounted to the outer housing 51 for allowing a syringe needle to inject medicine int the storage cavity 511.

The outer housing 51 serves as the main structural component, enclosing the internal parts of the medicine storage assembly 50. The storage cavity 511 within the outer housing 51 is a designated space for storing the medicine liquid before it is atomized. This centralized storage design reduces the complexity of handling and transferring medicine, potentially improving the safety and accuracy of the dosage.

The medicine liquid transporting element 52, which can be specified to be cotton, plays a significant role in the delivery system by acting as an efficient medium for transporting the medicine liquid from the storage cavity 511 to the atomization assembly 60. Cotton is a porous and absorbent material, ideal for drawing and holding liquid, and its use as a transporting element offers several key advantages:

Cotton fibers have natural capillary properties that allow them to wick liquid upward from the storage cavity 511. This capillary action ensures a consistent flow of medicine to the atomization assembly 60, supporting continuous atomization without interruption. The efficient liquid transfer helps maintain a steady mist output, which is essential for treatments that require consistent dosing over time, like respiratory therapy.

The high absorbency of cotton allows it to hold a significant volume of liquid in relation to its size. This property ensures that medicine can be retained within the cotton element, reducing the risk of leakage or spillage even when the atomizer is moved or tilted.

The absorbent nature of cotton acts as a temporary reservoir, minimizing waste by ensuring that only the necessary amount of liquid is transferred to the atomizer. This enhances dosing precision and reduces medicine waste.

The fiber density and absorption also help to control the liquid flow, reducing the chance of backflow from the atomization assembly 60 back into the storage cavity 511. This containment capability helps in preventing contamination of the remaining medicine in the storage area.

Backflow prevention is particularly beneficial for maintaining the purity of the remaining liquid, which is essential in medical devices where contamination could lead to reduced efficacy or increased health risks.

Cotton is soft and adaptable, allowing it to fit snugly within the storage cavity and maintain contact with the medicine liquid. This consistent fit prevents air bubbles or gaps that could disrupt the liquid flow. The flexibility of cotton ensures a reliable connection between the storage cavity 511 and the atomization assembly 60, making the medicine delivery process continual, smoother and more predictable.

In addition, fibrous structure of cotton can trap small particles, debris, or impurities in the medicine liquid, acting as a preliminary filter before the liquid reaches the atomization assembly. This filtration helps ensure that only purified medicine is atomized.

By filtering out impurities, the atomizer delivers cleaner, safer medicine mist to the patient, which is particularly important in medical treatments where respiratory systems are sensitive to contaminants.

The cotton filter can prevent particulate matter from entering the atomization assembly 60, which can be susceptible to clogging or reduced functionality when exposed to foreign particles. Keeping these contaminants out helps maintain the efficiency of the atomization process and prevents wear or damage to the assembly. This protection ensures a longer lifespan for the atomization assembly 60, reducing maintenance requirements and providing consistent performance over time.

In medical applications, any contaminants in the medicine could potentially cause adverse reactions, especially when the atomized medicine is inhaled. The cotton filter adds a layer of safety by removing potential contaminants that might cause irritation, infection, or other negative effects. The filtration function promotes patient safety by ensuring a cleaner, safer medicine mist, which is critical for patients with respiratory issues or compromised immune systems.

The cotton also brings a significant functional advantage to the atomizer by enabling consistent delivery of medicine liquid independent of the device's orientation. Unlike designs that rely on gravity to transport the liquid, the cotton material's absorbent properties ensure continuous medicine delivery, regardless of whether the atomizer is standing, tilted, or even upside down.

By eliminating the need for the atomizer to remain upright, the cotton transporting element allows users greater flexibility in how they hold and position the device. Patients can use the atomizer in a range of positions that are most comfortable, convenient, or suited to their condition, without worrying about medicine flow interruption.

The medicine injection structure 53 allows for easy injection of medicine into the storage cavity using a syringe. By having a dedicated injection point, users can quickly refill the atomizer without needing to open the main assembly, which can reduce exposure to contaminants and simplify the refilling process.

In this embodiment, the medicine injection structure 53 comprises an injection lid 531 mounted to a top of the outer housing 51 to provide a dedicated port for refilling, a connecting element 532 connected to the injection lid 531 and a sealing pin 533 vertically extended from the connecting element 532 for being inserted into the injection lid 531 for sealing the outer housing 51.

The medicine liquid transporting element 52 of this embodiment comprises a body portion 521 and an end portion 522 extended from the body portion 521, in this embodiment, the end portion 522 is extended through an opening 512 communicated to the storage cavity 511 of the outer housing 51 for feeding the medicine liquid to the atomization assembly 60. A diameter of the end portion 522 is smaller than or equal to a diameter of the body portion 521, in this embodiment, the diameter of the end portion 522 is smaller than the diameter of the body portion 521.

The medicine storage assembly 50 further comprises a sealing member 54 which is a sealing ring provided at the opening 512 and wound around the end portion 522 of the medicine liquid transporting element 52 to provide a sealing effect.

The atomization assembly 60 comprises a base 61, a connecting tube 62 and an atomizing element 63. The atomizing element 63 is mounted on the base 61, and the connecting tube 62 is detachably coupled to the base 61 for supporting the outer housing 51 of the medicine storage assembly 50. The connecting tube 62 can be connected to an air feeding tube, the base 61 can be connected to a a trachea cannula or a breathing mask for feeding the medicament mist to the user. In this embodiment, the connecting tube 62 is coupled between the outer housing 51 and the base 61.

In this embodiment, the end portion 522 of the medicine liquid transporting element 52 is extended into the connecting tube 62 for delivering medicine liquid to the atomizing element 63.

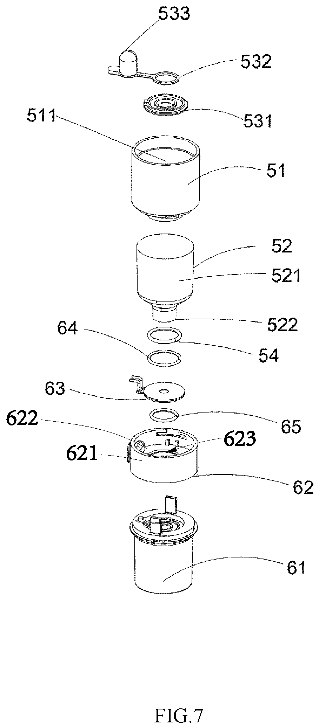

As shown in Fig. 7 of the drawings, when the medicine storage assembly 50 is mounted on the atomization assembly 60, the end portion 522 of the medicine liquid transporting element 52 is aligned with the atomizing element 63 and the distance between the end portion 522 of the medicine liquid transporting element 52 and the atomizing element 63 is preferred to be less than 0.3 mm. This close proximity minimizes the potential for medicine loss due to evaporation or dispersion and ensures that the maximum amount of medicine liquid reaches the atomizing element 63 for conversion into a mist. The precise distance also helps control the flow of liquid, promoting consistent droplet size in the mist, which can improve the delivery of the medicine to the respiratory system of the user.

The connecting tube 62 comprises a tube wall 621, a ring wall 622 extended from an inner side of the tube wall 621 and defines a penetrating hole 623 for allowing the end portion 522 of the medicine liquid transporting element 52 to pass therethrough. The sealing element 54 is provided at the top of the ring wall 622.

The connecting tube 62 comprises a first connecting end portion 624 for connecting to an air feeding tube and a second connecting end portion 625 for connecting to an air recycling tube. Accordingly, air can be fed into the connecting tube 62 and pass through the end portion 522 of medicine liquid transporting element 52, and then reach the atomizing element 63, so as to mix with the mist generated by the atomizing element 63 and finally enter a cavity 611 through a mist outlet 612 of the base 61.

The atomization assembly 60 further comprises a first sealing ring 64 which is mounted at a bottom of the ring wall 622 and is positioned above the atomizing element 63 around the end portion 522 of the medicine liquid transporting element 52 , and a second sealing ring 65 which is mounted at a top of the base 61 under the atomizing element 63.

In this embodiment, any suitable detachable connecting manner can be employed to couple the connecting tube 62 with the base 61, such as a snap-fit mechanism, a thread engaging connection, and a magnetic coupling connection. Any suitable detachable connecting manner also can be employed to couple the connecting tube 62 with the outer housing 51 of the medicine storage assembly 50, such as a snap-fit mechanism, a thread engaging connection, and a magnetic coupling connection.

The nebulized medicine can be replaced simply by replacing the medicine storage assembly 50, eliminating the cleaning and disinfection process of the previous plan. The operation is simple, and the medicine can be prepared in advance, which improves the work efficiency of medical staff and reduces cross-infection in the hospital.

The atomizing element 63 is a nebulizer atomizing sheet which is a vibrating mesh with pores. Through the high-frequency resonance of the nebulizer, naturally floating medicine mist is generated.

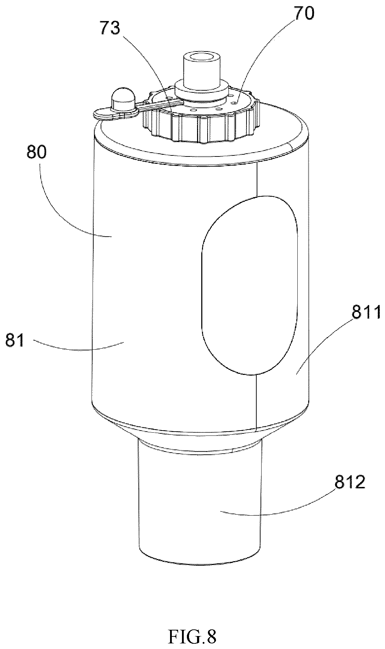

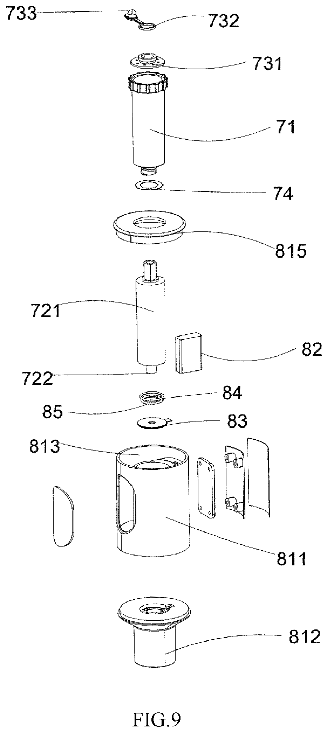

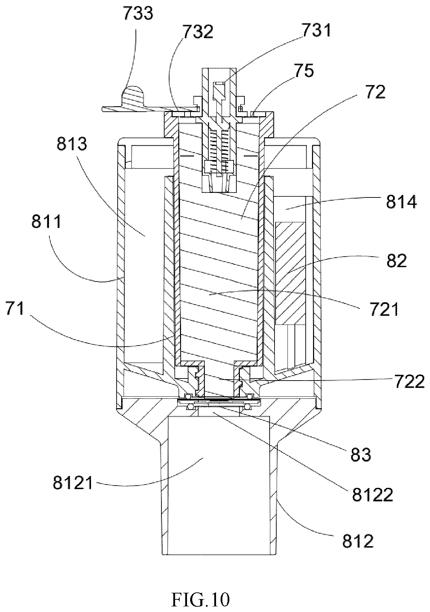

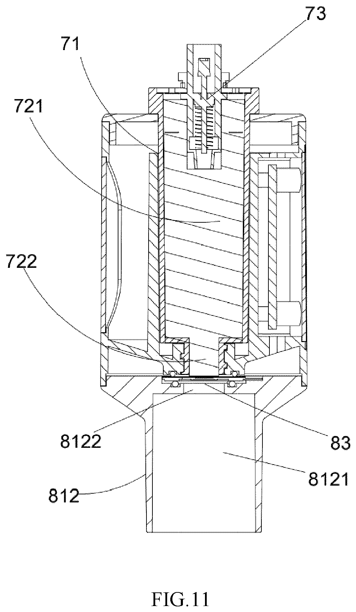

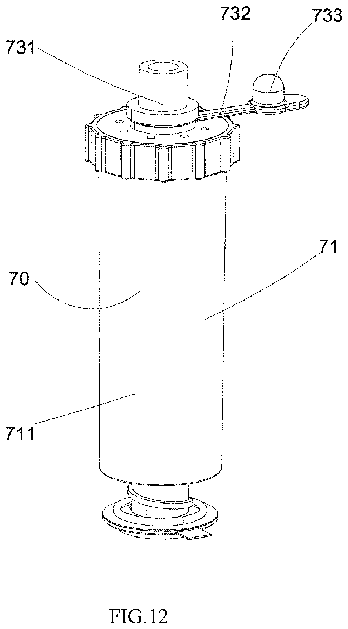

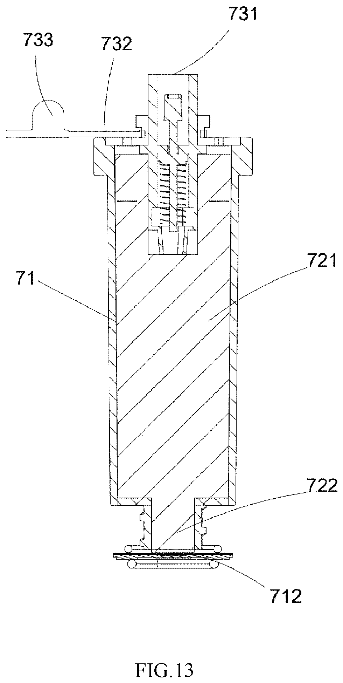

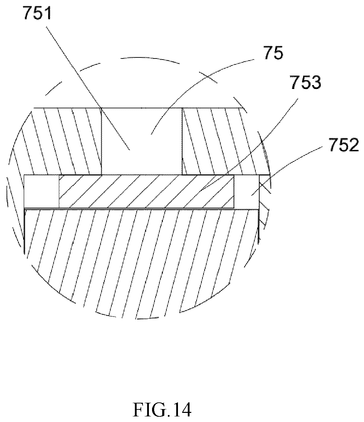

Referring to Figs. 8 to 14 of the drawings, an atomizer according to a third preferred embodiment of the present invention is illustrated. The atomizer comprises a medicine storage assembly 70 and an atomization assembly 80. In this embodiment, the medicine storage assembly 70 is detachably disposed in the atomization assembly 80.

More specifically, the medicine storage assembly 70 comprises a storage housing 71 having a storage cavity 711, a medicine liquid transporting element 72 disposed in the storage cavity 711 and a medicine injection structure 73.

Similarly, the medicine injection structure 73 comprises an injection lid 731 mounted to a top of the storage housing 71 to provide a dedicated port for refilling, a connecting element 732 connected to the injection lid 731 and a sealing pin 732 vertically extended from the connecting element 732 for being inserted into the injection lid 731 for sealing the storage housing 71.

The medicine liquid transporting element 72 can be embodied as a cotton comprising a body portion 721 and an end portion 722 extended from the body portion 721, in this embodiment, the end portion 722 is extended through an opening 712 communicated to the storage cavity 711 of the storage housing 71 for feeding the medicine liquid to the atomization assembly 80. A diameter of the end portion 722 is smaller than or equal to a diameter of the body portion 721.

The medicine storage assembly 70 further comprises a sealing member 74 which is a sealing ring provided at the opening 712 and wound around the end portion 722 of the medicine liquid transporting element 72 to provide a sealing effect.

The atomization assembly 80 comprises a fixing housing 81, a control unit 82 and an atomizing element 83 electrically connected to the control unit 82. The fixing housing 81 comprises a housing body 811 and a base 812 connected to the housing body 811 for mounting the atomizing element 83.

In this embodiment, the storage housing 71 is disposed into an inner cavity 813 of the housing body 811 of the fixing housing 81 so that the storage housing 71 is provided in the fixing housing 81. The fixing housing 81 has a side chamber 814 between the storage housing 71 and the housing body 811 for receiving the control unit 82.

The atomizing element 83, which is preferred to be an atomizing sheet with pores, is mounted at a top of the base 812 and is aligned with the end portion 722 of the medicine liquid transporting element 72, a distance between the atomizing element 83 and the end portion 822 of the medicine liquid transporting element 72 can be less than 0.3 mm.

The atomization assembly 80 may further comprise a first sealing ring 84 which is mounted at a bottom of the housing body 811 and is positioned above the atomizing element 83 around the end portion 722 of the medicine liquid transporting element 72, and a second sealing ring 85 which is mounted at the top of the base 812 under the atomizing element 83.

The base 812 has a cavity 8121 and a mist outlet 8122 under the atomizing element 83, the atomizing element 83 is vibrating to generate medicament mist which is discharged into the cavity 8121 through the mist outlet 8122. The base 812 can be connected to a a trachea cannula or a breathing mask for feeding the medicament mist to the user.

In this embodiment, the medicine storage assembly 70 can be replaced, so that the medicine that needs to be atomized can be replaced conveniently and quickly, and the control module can be retained and reused, saving patients' expenses.

Any suitable detachable connecting manner can be employed to couple the housing body 811 with the storage housing 71, such as a snap-fit mechanism, a thread engaging connection, and a magnetic coupling connection.

The storage housing 71 is further provided with an air exhaust structure 75 having an exhaust hole 751 located at the top wall of the storage housing 71, an air retention area 752 located at the exhaust hole 751, and comprises a water-blocking and breathable membrane 753 located between the exhaust hole 751 and the air retention area 752. The exhaust hole 751 exhausts excess air during medicine injection to balance internal pressure, and the water-blocking and breathable membrane 753 prevents medicine volatilization or leakage, and also prevents external contaminants from entering and contaminating the medicine solution.

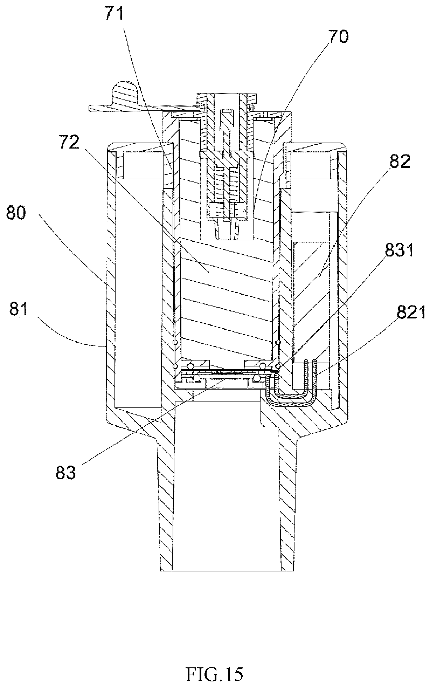

Referring to Fig. 15 of the drawings, as an alternative mode of the above third preferred embodiment, the atomizing element 83 comprises two first connecting terminals 831 while the control unit 82 comprises two second connecting terminals 821, the two first connecting terminals 831 can be respectively detachably and electrically connected to the two second connecting terminals 821 when the medicine storage assembly 70 is assembled into the fixing housing 81 of the atomization assembly 80.

In this embodiment, when the atomizing element 83 is detached from the control unit 82, the control unit 82 still can be reused to be electrically connected to a new atomizing element 83.

## Claims

1. An atomizer, comprising:
a medicine storage assembly (70); and
an atomization assembly (80), wherein said medicine storage assembly (70) is detachably coupled to said atomization assembly (80), wherein said medicine storage assembly (70) comprises a storage housing (71) having a storage cavity (711), and a medicine liquid transporting element (72) disposed in said storage cavity (711) of said storage housing (71), wherein said atomization assembly (80) comprises a fixing housing (81) and an atomizing element (83) disposed in said fixing housing (81) to align with said medicine liquid transporting element (72) in a manner that said medicine liquid transporting element (72) is arranged to deliver medicine liquid to said atomizing element (83), wherein said storage housing (71) is detachably coupled within said fixing housing (81), **characterised in that**:
said storage housing (71) is further provided with an air exhaust structure (75) having an exhaust hole (751) located at a top of said storage housing (71) and an air retention area (752) located adjacent to said exhaust hole (751), wherein said air exhaust structure (75) further comprises a water-blocking and breathable membrane (753) located between said exhaust hole (751) and said air retention area (752).

2. The atomizer according to claim 1, wherein said medicine storage assembly (70) further comprises a medicine injection structure (73) which comprises an injection lid (731) mounted to a top of said storage housing (71) for refilling, a connecting element (732) connected to said injection lid (731) and a sealing pin (733) extended from said connecting element (732) for being inserted into said injection lid (731).

## Patentansprüche

1. Ein Zerstäuber, umfassend:
eine Medikamentenaufbewahrungseinheit (70) ; und
eine Zerstäubungsanordnung (80) , wobei die Medikamentenaufbewahrungseinheit (70) lösbar mit der Zerstäubungsanordnung (80) verbunden ist , wobei die Medikamentenaufbewahrungseinheit (70) ein Aufbewahrungsgehäuse (71) mit einem Aufbewahrungsraum (711) und ein Medikamentenflüssigkeitstransportelement (72) umfasst, das in dem Aufbewahrungsraum (711) des Aufbewahrungsgehäuses (71) angeordnet ist , wobei die Zerstäubungsanordnung (80) ein Befestigungsgehäuse (81) und ein Zerstäubungselement (83) umfasst , das in dem Befestigungsgehäuse (81) so angeordnet ist , dass es mit dem Medikamentenflüssigkeitstransportelement (72) so ausgerichtet ist, dass das Medikamentenflüssigkeitstransportelement (72) die Medikamentenflüssigkeit an das Zerstäubungselement (83) abgibt , wobei das Aufbewahrungsgehäuse (71) lösbar mit dem Befestigungsgehäuse (81) verbunden ist , **dadurch gekennzeichnet , dass**:
Das genannte Speichergehäuse (71) ist ferner mit einer Luftablassstruktur (75) versehen , die eine Ablassöffnung (751) an der Oberseite des Speichergehäuses (71) und einen Luftrückhaltebereich (752) neben der Ablassöffnung (751) aufweist , wobei die Luftablassstruktur (75) ferner eine wasserabweisende und atmungsaktive Membran (753) umfasst , die sich zwischen der Ablassöffnung (751) und dem Luftrückhaltebereich (752) befindet.

2. Der Zerstäuber nach Anspruch 1, wobei die Medikamentenaufbewahrungseinheit (70) ferner eine Medikamenteninjektionsstruktur (73) umfasst, die einen Injektionsdeckel (731) zur Nachfüllung auf der Oberseite des Aufbewahrungsgehäuses (71) aufweist , ein mit dem Injektionsdeckel ( 731) verbundenes Verbindungselement ( 732) und einen von dem Verbindungselement (732) abstehenden Dichtungsstift ( 733) zur Einführung in den Injektionsdeckel (731).

## Revendications

1. Un atomiseur, comprenant :
un ensemble de stockage de médicaments (70) ; et
un ensemble d'atomisation (80) , dans lequel ledit ensemble de stockage de médicament (70) est couplé de manière amovible audit ensemble d'atomisation (80) , ledit ensemble de stockage de médicament (70) comprenant un boîtier de stockage (71 ) doté d'une cavité de stockage (711) , et un élément de transport de liquide médicamenteux (72) disposé dans ladite cavité de stockage (711) dudit boîtier de stockage (71) , ledit ensemble d'atomisation (80) comprenant un boîtier de fixation (81) et un élément d'atomisation (83) disposé dans ledit boîtier de fixation (81) de manière à s'aligner avec ledit élément de transport de liquide médicamenteux (72) de façon à ce que ledit élément de transport de liquide médicamenteux (72) soit agencé pour acheminer le liquide médicamenteux audit élément d'atomisation (83) , ledit boîtier de stockage (71) étant couplé de manière amovible à l'intérieur dudit boîtier de fixation (81) , **caractérisé en ce que** :
ledit boîtier de stockage (71) est en outre doté d'une structure d'évacuation d'air (75) comportant un orifice d'évacuation (751) situé sur le dessus dudit boîtier de stockage (71) et une zone de rétention d'air (752) située à proximité dudit orifice d'évacuation (751) , ladite structure d'évacuation d'air (75) comprenant en outre une membrane étanche à l'eau et respirante (753) située entre ledit orifice d'évacuation (751) et ladite zone de rétention d'air (752).

2. L'atomiseur selon la revendication 1, dans lequel ledit ensemble de stockage de médicament (70) comprend en outre une structure d'injection de médicament ( 73) qui comprend un couvercle d'injection (731) monté sur le dessus dudit boîtier de stockage (71) pour le remplissage, un élément de connexion (732) relié audit couvercle d'injection (731) et une goupille d'étanchéité (733) s'étendant à partir dudit élément de connexion (732) pour être insérée dans ledit couvercle d'injection (731).
